# EUROPEAN PATENT APPLICATION

(11) **EP 1 208 754 A1**
(43) Date of publication of application: **29.05.2002**
(21) Application number: 00124384.9
(22) Date of filing: 21.11.2000
(51) Int. Cl.: A23L 1/22, A23P 1/04, A23P 1/02, A61K 7/46, B01J 13/04

(54) **Particulate material**

(71) Applicant: Givaudan SA, 1214 Vernier-Genève (CH)
(72) Inventor: Schudel, Markus, 8052 Zürich (CH); Quellet, Christian, 2502 Biel (CH); Taschi, Marc, 8400 Winterthur (CH); Bouwmeesters, Johnny, 8618 Oetwil am See/ZH (CH)
(74) Representative: Patentanwälte Schaad, Balass, Menzl & Partner AG

(57) **Abstract**

The present invention relates to a particulate material comprising at least 25% by weight of a volatile substance having an inner part covered by an outer part, the inner part having core particles embedded in a matrix, wherein the core particles comprise a solid, water- or alkali soluble or swellable, non-porous core particle substance S₁ and core particle inclusions I₁ comprising a core particle volatile substance V₁ and the matrix comprises a hydrophilic matrix substance S₂ and matrix inclusions I₂ comprising a matrix volatile substance V₂.

## Description

The present invention relates to a particulate material comprising at least 25% by weight of a volatile substance and to a process for preparing said particulate material.

A principal strategy currently employed in imparting odors into consumer products is the admixing of a volatile substance such as a fragrance directly into the product. There are, however, several drawbacks to this strategy. The fragrance material can be too volatile, resulting in fragrance loss during manufacturing, storage and use. Many fragrance materials are also unstable over time. This again results in loss of perfume during storage.

In many consumer products it is desirable that the fragrance is released throughout the whole application cycle. Further in many consumer products it is desirable that the fragrance is released slowly over time from substrates on which said fragrance has been deposited. Since the most volatile fragrances, or "top notes" are responsible for the "fresh feeling" consumers experience, it is desirable that in addition to the less volatile fragrances of a fragrance composition also the more volatile fragrances are slowly released.

The use of starch and modified starches to encapsulate volatile substances by spray drying technologies is disclosed in US 3971852 and US 5508259. Starch particles exhibit an immediate, burst-like release after contacted with water.

In WO 99/38945 water dispersible granulate for laundry applications are disclosed. These granules are produced either by spray drying or lyophilisation, using specially selected encapsulation material such as polyelectrolytes and polypeptides of plant and synthetic origin. However, said particles are rather small and where the use of polyelectrolytes is expected to increase the sensitivity to humidity.

In WO 99/38946 the matrix material of the granulated material is replaced by an alkali-soluble polymer derived from emulsion polymerization, said particles being dispersed in a water-soluble or water dispersible dry organic compound. However, granulates and alkali-soluble polymers are very sensitive to alkaline conditions.

WO 00/12669 discloses a particulate composition for incorporation in washing powder formulations comprising high amounts of highly amorphous silica and at least 30 % by weight of perfume. A substantial disadvantage is the very fast, almost uncontrolled, release of perfuming ingredients during the very first part of a normal washing cycle. Additionally, it can be expected by those skilled in the art that such particles will be quite sensitive to humidity leading to low storage performance in terms of perfume fragrance retention.

WO 99/45091 discloses a method for producing highly dosed fragrant compounds called perfumed beads. Fast disintegration of the detergent forms, including the perfumed beads, is desired and no controlled release happens during the wash cycle.

EP 0 070 719 discloses a process of granulation yielding particles with a rather broad particle size distribution.

In WO 97/16078 the load of volatile substances into a granulate is claimed to be limited to a maximum of 25 %.

Therefore, the amount of perfume is limited until now by the requirement of fast disintegration in the wash liquor, acceptable stability during storage and/or high mechanical resistance during conveying, mixing with detergent powders or during tablet manufacturing.

There remains a strong need for a good particulate material, which allows high loads of volatile substances, minimal loss of said volatile substances during the granulation process, excellent protection against evaporation and chemical degradation at high relative humidity, and optimal release of the said volatile substances in aqueous media, especially in wash liquor such as those encountered in laundry.

The object of the present invention is therefore to provide a particulate material with high protection of volatile substances under high humidity conditions.

A further object is to provide a particulate material, which delivers the volatile substances on a controlled manner in aqueous media.

A further object is to provide a particulate material which delivers the volatile substance on a controlled manner during washing in order to enhance the deposition of these volatile substances on substrates, in particular of volatile olfactory substances on fabrics, compared to detergents having no fragrance-containing particulate material.

A further object of the present invention is to provide a particulate material, which enhances the long lastingness of the volatile olfactory substances on the dry fabrics after rinse and drying stages.

A further object of the present invention is to provide a particulate material for volatile substances having secondary benefits such as high mechanical stability and good flowing performance, e.g. dust-free and free flowing, and visual effects like color.

A further object of the present invention is to provide a cost-effective process for preparing a particulate material. On another hand the granulate should be dust-free and has a narrow particle size distribution.

A high protection and a controlled release of volatile substances, especially of organoleptic substances, can be obtained by particulate material according to the present invention, comprising at least 25% and even more than 40% by weight of a volatile substance, having an inner part which is covered by an outer part. Said inner part consists of core particles embedded in a matrix, wherein the core particles comprise a solid, water- or alkali-soluble, or swellable, non-porous core particle substance S₁ and up to 80% by weight of core particle inclusions I₁ comprising a core material volatile substance V₁. The matrix comprises a hydrophilic matrix substance S₂ and up to 75% by weight of matrix inclusions I₂ comprising a matrix volatile substance V₂. Said outer part consists of a water-insoluble or sparingly soluble or water repellent or swellable substance covered on the particulate material covered by the inner part.

In a preferred embodiment of the present invention the core particles comprise additionally a core particle emulsifier E₁ and the matrix comprise additionally a matrix emulsifier E₂. The particulate material according to the present invention has a size between 0.1 and 2 mm. For powder application a size of particulate material between 0.1 and 1.5 mm, for tablets a size of particulate material between 0.1 and 1 mm is preferred. The core particles have particle sizes between 0.05 and 1.0 mm, preferably between 0.1 mm and 0.4 mm, most preferably between 0.1 mm and 0.3 mm.

The particulate material according to the present invention has a high bulk density of 0.3 to 1.5 g /cm³, preferably 0.5 to 0.9 g/cm³, is mechanically stable, free-flowing and nearly dust-free. Within the context of the present invention the term 'dust-free product' is related to a particulate material exhibiting a fine particle fraction of < 5 % for particle sizes < 0.1 mm.

The core particle inclusions I₁ comprise 50 to 100% by weight of the core material volatile substance V₁ and the matrix inclusions I₂ comprise 50 to 100% of the matrix volatile substance V₂.

In a preferred embodiment core particle inclusions I₁ comprise up to 50% by weight of a core particle carrier C₁ for the core material volatile substance V₁ and the core material volatile substance V₁. The core material volatile substance V₁ can be deposited in or on a core particle carrier C₁. The core particle inclusions I₁ have a size ranging from 0.1 to 20 µm, preferably from 0.1 to 5 µm. In a further preferred embodiment of the present invention the matrix inclusions I₂ comprise up to 50% by weight of a matrix carrier C₂ for the matrix volatile substance V₂ and the matrix volatile substance V₂. The matrix volatile substance V₂ can be deposited in or on the matrix carrier C₂. The matrix inclusions I₂ have a size ranging from 0.1 to 20 µm, preferably from 0.1 to 5 µm. Most preferred are embodiments wherein the core material volatile substance V₁ is deposited in or on the core particle carrier C₁ and the matrix volatile substance V₂ is deposited in or on the matrix carrier C₂.

The core particle substance S₁ and the matrix substance S₂ are hydrophilic materials and may be selected from:
(1) Carbohydrates such as (i) monosaccharides; (ii) oligosaccharides; (iii) polysaccharides; (iv) starches including modified starches and hydrolysates; (v) hydrogenates of (i-iv). Both linear and branched carbohydrate chains can be used. In addition, chemically modified starches and poly-/oligo-saccharides may be used including the addition of hydrophobic moieties of the form of alkyl, aryl, etc.;(vi) sucrose esters, glycerol esters like tripalminate, tristearate, palmito stearate, behenate, etc.
(2) Natural or synthetic gums such as alginate esters, carrageenan, xanthanes, agar-agar, pectines, pectic acid, and natural gums such as gum arabic, gum tragacanth and gum karaya.
(3) Chitin and cationic polysaccharides like chitosan derivatives, salts and mixtures thereof.
(4) Cellulose and cellulose derivatives such as (i) cellulose acetate and cellulose acetate phthalate; (ii) hydroxy methyl propyl cellulose; (iii) carboxy methyl cellulose; hydroxy propyl methyl cellulose phthalate, hydroxy propyl methyl cellulose acetate succinate and their salts; (iv) all enteric/aquateric coatings and mixture thereof.
(5) Polypeptides such as (i) plant proteins like hydrolyzed oat proteins; (ii) animal proteins like gelatin, collagen, elastin, keratin, etc., and their hydrolyzed or quaternized derivatives; (iii) proteins of synthetic origin like homopolymers and copolymers produced by polycondensation of amino acids like aspartamic and glutamic acid, glycine, alanine, leucine, etc.
(6) Synthetic water soluble or partially water soluble, or degradable polymers such as (i) vinyl polymers like poly(vinyl alcohol), poly(vinyl alcohol-co-vinyl acetate). copolymers, poly(vinyl alcohol-co-vinyl acetate-co-sodium vinyl sulfonate) copolymers; poly(vinyl pyrrolidon) and copolymer derivatives such as poly(vinyl pyrrolidon-co-vinyl acetate), poly(vinylpyrrolidon-co-vinylalcohol), poly(vinylpyrrolidon-co-styrene); (ii) alkali-soluble polymers like poly(acrylic acid), poly(maleic acid), poly(alkyl(meth)acrylate-co-(meth)acrylic acid) copolymer, poly(acrylic acid-co-maleic acid)copolymer, poly(acrylamide), poly(methacrylic acid), poly((meth)acryl amide) as well as other acrylic homo- and copolymers thereof; (iii) polyoxides, polyethers and poly(ethyleneimine) like poly(ethyleneoxide), poly(propyleneoxide), poly(vinylmethylether), poly-(ethyleneimine), and their mixture and salts; (iv) poly(vinyloxazolidone)and poly(vinyl methyl oxazolidone), partially esterified copolymers of methyl vinyl ether and maleic anhydride like poly(vinylether-co-maleic anhydride) and its acid forms; (v) silicone polyglucoside derivatives such as poly (dimethicone-co-ethoxy glucoside) copolymer derivatives or mixtures thereof.
(7) Polyelectrolytes such as poly (ethylene imine) derivatives or poly (acrylic acid) derivatives or mixture thereof.
(8) Biodegradable (bio)polymers like (i) polyesters, especially lactic, glycolic, hydroxybutyric acid derivatives such as poly (D,L-lactide), poly (L-lactide), poly (L-lactide-co-glycolide), poly(D,L-lactide-co-glycolide), poly (D,L-lactide-co-glycolide-D-glucose), poly (hydroxybutyric acid) and further derivatives, copolymers or mixtures thereof; (ii) water soluble or water dispersible forms of poly (3-hydroxy butyrate) derivatives; (iii) lignin derivatives such as lignosulphonates, oxylignins and kraft lignins and further derivatives or mixture thereof.
(9) Plasticizers such as triacetin, triethylcitrate, polyethylene glycol, diethyl phthalate, tributyl citrate, glycerin or other conventional plasticizers and their mixtures.
(10) Inorganic materials such as silicates, phosphates, borates, acetates, clay minerals, zeolites in colloidal or standard form.

In a preferred embodiment of the present invention the core particles comprise a core particle emulsifier E₁ and/or the matrix comprises a matrix emulsifier E₂. The core particle and matrix emulsifier can be a mixture of octenyl succinate substituted starch or a non substituted starch like Capsul® , Hi-Cap 100® , sucrose esters like Sisterna PS750 or synthetic polymer like poly (vinyl alcohol) such as Mowiol® , or derivatives in combination with suitable surface active agents. Instead of using only a mixture of octenyl succinate substituted starch or a non substituted starch, long chain glycosides such as sucrose ester, optionally combined with short chain emulsifiers, such as sorbates, sorbitan esters and their ethoxylated derivatives, etc. Furthermore, a blend of said modified starch free emulsifiers and modified starch octenyl succinate emulsifiers can be used.

In a preferred embodiment the solid, water- or alkali-soluble or swellable, non-porous substance S₁ is glassy and not sticky and has a glass-transition temperature Tg which is higher then room temperature.

The selection of the core particle material S₁ is done by emulsifying an emulsifier E₁ with the core particle material S₁ the core particle volatile substance V₁ and water in such a way that the core particle inclusion I₁ is dispersed in the form of small droplets or particles in the aqueous phase and that the interface between said droplets or particles and the aqueous phase is saturated with said emulsifier E₁ in order to get a stable oil-in-water feed emulsion for spray drying. This is achieved for example at a core particle inclusion I₁ to effective emulsifier E₁ mass ratio of lower than 25:2 (w/w) for typical commercially available modified starch emulsifiers. This mass ratio of 25:2 (w/w) has to be selected as a higher limit in order to prevent extensive surface oil formation and thus to achieve optimal initial fragrance retention during spraying in dried particles comprising more than 30% by weight of a volatile substance.

In order to form a particulate material with a high density, narrow particle size distribution, high volatile substance loading with maximal volatile retention, the matrix substance S₂ must show the right balance of tack and water content under agglomeration conditions. As the latter properties are difficult to measure directly in the fluid bed, equilibrium measurements on cast binder films, as described in EXAMPLE 1, are preferred. In particular, data obtained on thin cast binder films after equilibration at 70% relative humidity and 25°C can be advantageously used to select the right binder composition according to the present invention. In the case of the matrix, a matrix inclusion I₂ to effective emulsifier E₂ mass ratio of lower than 25:2 (w/w) for typical commercially available modified starch emulsifiers is preferred as well.

It has, therefore, been found that ideal matrices (i.e. matrix volatile substance V₂, optionally matrix carrier C₂, matrix emulgator E₂ and matrix substance S₂, form 100 +/- 10 µm thick cast films with an equilibrium water content at 70% relative humidity lower than 10% by weight, preferably lower than 5% by weight, and a rolling-ball tack value ranging between 4 cm and 20 cm, preferably between 6 cm and 15 cm, and most preferably between 8 and 12 cm.

The rolling ball tack measurement is generally believed to yield a convenient picture of the ability of matrix substances to adhere readily to a surface, i.e. to establish an adhesive bond with a surface within a time scale not exceeding 1 second. The rolling ball tack value is inversely proportional to the real tack. Hence, the lowest the rolling-ball tack, the highest the real tack and the fastest the formation of an adhesive bond between the matrix substance and the surface. For comparison, conventional adhesive tapes such as those used in offices and household have a rolling ball tack value of 10 to 12 cm, while low temperature adhesives have a rolling ball tack value not exceeding 2 to 5 cm. A description of the rolling ball tack method can be found in *Tack Rolling Ball Test Method PSTC 6,* available from Pressure Sensitive Tape Council, page 19-64, 12. edition, August 1999. For everyone skilled in the art of adhesive formulation, a convenient alternative to the rolling ball tack measurement is furnished by using the so-called "finger tip method", i.e. by applying and removing fast the tip of a finger onto the adhesive and estimating the "grip" of the adhesive. Matrices substances S₂ forming cast films with a finger tip grip comparable to that of a conventional adhesive tape, after equilibration at 70% relative humidity and 25°C, are particularly suitable for the sake of the present invention.

The. core material volatile substances V₁, the matrix volatile substances V₂ and the outer part volatile substance V₃ can be selected from olfactory compounds, bio-active materials like insect, cockroach and acarian repellents, bactericides and bacteriostatic agents, cosmetic agents or any organic volatile or reactive liquid. The core material volatile substances V₁, matrix volatile substance V₂ and the outer part volatile substance V₃ can be of the same or of a different chemical nature. The expression olfactory compound stands for fragrance or flavor, a masking agent or a precursor thereof. The general expression volatile substance comprises the core material volatile substance V₁, the matrix volatile substance V₂ and the outer part volatile substance V₃. The core material volatile substance V₁ comprises one or more components. The matrix volatile substance V₂ as well as the outer part volatile substance V₃ comprise one or more components as well.

The volatile substance which can be used for particulate materials according to the present invention, may be natural products such as essential oils, absolutes, resinoids, resins, concretes etc., and synthetic perfume components such as hydrocarbons, alcohols, aldehydes, ketones, ethers, acids, acetals, ketals, nitriles, etc., including saturated and unsaturated compounds, aliphatic, carbocyclic and heterocyclic compounds. Other examples of odorant compositions which may be used are described in H 1468 (United States Statutory Invention Registration).

The existence of several distinct hydrophobic inclusion. phases that allows the encapsulation of distinct olfactory components within the same delivery system is a substantial advantage. The clear benefit of such a configuration in terms of volatile substance retention during process (i.e. initial retention) and storage (i.e. aged retention) is that the volatile substance can be split into two or more distinct fractions, which, in turn, can be included in two or more distinct phases. Hence, volatile substances with poor initial retention can be included preferentially in core particle inclusions I₁, as V₁, while the bulk of other less critical matrix volatile substances V₂ can be included in the matrix inclusions I₂. In a preferred embodiment olfactory compositions with different hedonic attributes or different physical properties can be dispersed in the core particles, and in the matrix and optionally in the outer part. The selection of core material volatile substances V₁ and matrix volatile substance V₂ is also part of the present invention.

In order to obtain high loads of volatile substances and maximal initial retention during process, the volatile substance is preferentially divided into two distinct compositions. In a preferred embodiment the core material volatile substance V₁ comprises at least 70 % of fragrance components with a loss factor ***f***_{**L**} higher than 5 Pa ppm (500 µg/l ppm), preferably higher than 10² Pa ppm (10⁴ µg/l ppm), most preferably higher than 10⁴ Pa ppm (10⁶ µg/l ppm), where said loss factor ***f***_{**L**} is related to the losses during drying and is defined by the product of the pure component vapor pressure (in Pa) and the water solubility (in ppm) and given therefore in values of [Pa ppm].

As an example, a non-limiting list of fragrance components is given which are preferably alone or in combination present as core particle volatile substance V₁ in the inclusions I₁ of the core particles, following the preferred selection described above (***f***_{L} higher than 10² Pa ppm):

2-Methyl Pyrazine, Acetaldehyde phenylethyl propyl acetal, Acetophenone, Alcohol C6 (in the following the notation Cn comprises all substances having n carbon atoms and one hydroxyl function), Alcohol C8, Aldehyde C6 (in the following the notation Cn encompasses all isomers having n carbon atoms and one aldehyde function), Aldehyde C7, Aldehyde C8, Aldehyde C9, Nonenylic Aldhyde, Allyl Amyl Glycolate, Allyl Caproate, Amyl Butyrate, Aldehyde anisique, Benzaldehyde, Benzyl Acetate, Benzyl Acetone, Benzyl Alcohol, Benzyl Butyrate, Benzyl Formate, Benzyl Iso Valerate, Benzyl Methyl Ether, Benzyl Propionate, Bergamyl Acetate, Butyl Acetate, Camphor, 3-methyl-5-propyl-2-cyclohexenone, Cinnamic Aldehyde, Cis-3-Hexenol, Cis-3-Hexenyl Acetate, Cis-3-Hexenyl Formate, Cis-3-Hexenyl Iso Butyrate, Cis-3-Hexenyl Propionate, Cis-3-Hexenyl Tiglate, Citronellal, Citronellol, Citronellyl Nitrile, 2-hydroxy-3-methyl-2-Cyclopenten-1-one, Cuminic Aldehyde, Cyclal C, Acetic Acid (cycloheyloxy)-2-propenylester, Damascenone, Damascone Alpha, Damascone Beta, Decahydro Beta Napthyl Formate, Diethyl Malonate, Dihydro Jasmone, Dihydro Linalool, Dihydro Myrcenol, Dihydro Terpineol, Dimethyl Anthranilate, Dimethyl Benzyl Carbinol, Dimethyl Benzyl Carbinyl Acetate, Dimethyl Octenone, Dimetol, Dimyrcetol, Estragole, Ethyl Acetate, Ethyl Aceto Acetate, Ethyl Benzoate, Ethyl Heptoate, Ethyl Linalool, Ethyl Salicylate, Ethyl-2-Methyl Butyrate, Eucalyptol, Eugenol, Fenchyl Acetate, Fenchyl Alcohol, 4-Phenyl-2,4,6-trimethyl 1,3-dioxane, Methyl 2-octynoate, 4-Isopropylcyclohexanol, 2-sec-Butylcyclohexanone, Styralyl acetate, Geranyl nitrile, Hexyl Acetate, Ionone Alpha, Iso Amyl Acetate, Iso Butyl Acetate, Iso Cyclo Citral, Dihydroisojasmone, Iso Menthone, Iso Pentyrate, Iso Pulegol, cis-Jasmone, Laevo Carvone, Phenylacetaldehyde glycerylacetal, carbinic acid 3 -Hexenyl Methyl Ether, 1-methyl-cyclohexa-1,3-diene, Linalool, Linalool Oxide, 2-;ethyl Ethyl Ester Pentanoate, 2,6-Dimethyl-5-heptenal, Menthol, Menthone, Methyl Acetophenone, Methyl Amyl Ketone, Methyl Benzoate, Methyl Cinnamic Aldehyde Alpha, Methyl Heptenone, Methyl Hexyl Ketone, Methyl Para Cresol, Methyl Phenyl Acetate, Methyl Salicylate, Neral, Nerol, 4-tert-Pentyl-cyclohexanone, Para Cresol, Para Cresyl Acetate, Para Tertiary Butyl Cyclohexanone, Para Tolyl Aldehyde, Phenyl Acetaldehyde, Phenyl Ethyl Acetate, phenyl ethyl alcohol, phenyl ethyl butyrate, phenyl ethyl formate, phenyl ethyl iso butyrate, phenyl ethyl propionate, Phenyl Propyl Acetate, Phenyl Propyl Aldehyde, Tetrahydro-2,4-dimethyl-4-pentyl-furan, 4-Methyl-2-(2-methyl-1-propenyl)tetrahydropyran, 5-Methyl-3-heptanone oxime, Styralyl Propionate, Styrene Monomer, 4-Methylphenylacetaldehyde, Terpineol, Terpinolene, Tetrahydro Linalool, Tetrahydro Myrcenol, Trans-2-Hexenal, 4,7-Methano-lH-3A,4,5,6,7,7A-hexahydro-acetate, Viridine

As a further example, a non-limiting list of fragrance components is given which are preferably alone or in combination present as matrix volatile substance V₂ in the matrix inclusions I₂ following the preferred selection described above **(*f***_{**L**} lower than 10² Pa ppm):

Aldron, Ambrettolide, Ambroxan, Benzyl Cinnamate, Benzyl Salicylate, Boisambrene Forte, Cedrol Crystals, Cedryl Acetate Crystals, Celestolide / Crysolide, Cetalox, Citronellyl Ethoxalate, Fixal, Fixolide, Galaxolide 50 DEP, Guaiacwood Acetate, Cis-3-Hexenyl Salicylate / LRT 17, Hexyl Cinnamic Aldehyde, Hexyl Salicylate, Iso E Super, Linalyl Benzoate FCC, Linalyl Cinnamate, Linalyl Phenyl Acetate, Methyl Cedryl Ketone, Moskene, Musk CPD, Musk Ketone, Musk Tibetine, Musk Xylol, Myraldyl Acetate, Nerolidyl Acetate, Novalide, Okoumal, Para-Cresyl Caprylate, Para Cresyl Phenyl Acetate Crystals, Phantolid Crystals, Phenyl Ethyl Cinnamate, Phenyl Ethyl Salicylate, Rose Crystals / Rosone, Sandela (Lin), Tetradecanitrile /LRG 1250, Thibetolide, Traseolide 100, Trimofix O.

As for fragrances, it may be of interest to encapsulate volatile and chemically sensitive flavor components. Flavored particles may provide a higher stability of the flavor during storage in food products and flavor burst upon chewing the food. As an example, a non-limiting list of flavor components is given which are preferably alone or in combination present as core material volatile substance V₁ in the inclusions I₁ of the core particles, following the preferred selection described above (***f***_{**L**} higher than 10² Pa ppm):

2-Methyl Pyrazine, Acetophenone Extra, Alcohol C6, Alcohol C8, Aldehyde C7 Heptylic, Aldehyde C8, Aldehyde C9, Allyl Caproate, Amyl Butyrate, Anisicaldhyde, Benzaldehyde, Benzyl Acetate, Benzyl Alcohol, Benzyl Butyrate, Benzyl Formate, Benzyl Iso Valerate, Benzyl Propionate, Butyl Acetate, Camphor, Cinnamic Aldehyde, Cis-3-Hexenol, Cis-3-Hexenyl Acetate, Cis-3-Hexenyl Formate, Cis-3-Hexenyl Propionate, Citronellal, Citronellol, Cuminic Aldehyde, Damascenone, Damascone Alpha, Damascone Beta, Diethyl Malonate, Dimethyl Anthranilate, Dimethyl Benzyl Carbinyl Acetate, Estragole, Ethyl Acetate, Ethyl Aceto Acetate, Ethyl Benzoate, Ethyl Heptoate, Ethyl Salicylate, Ethyl-2-Methyl Butyrate, Eucalyptol, Eugenol, Fenchyl Acetate, Fenchyl Alcohol, Methyl-2-octynoate, 2-sec-Butylcyclohexanone, Styralyl Acetate, Hexyl Acetate, Ionone Alpha, Iso Amyl Acetate, Iso Butyl Acetate, Iso Menthone, Jasmone Cis, Laevo Carvone, Linalool, Linalool Oxide, Melonal, Menthol, Menthone, Methyl Acetophenone, Methyl Amyl Ketone, Methyl Benzoate, Methyl Heptenone, Methyl Hexyl Ketone, Methyl Para Cresol, Methyl Phenyl Acetate, Methyl Salicylate, Neral, Nerol, Para Cresol, Para Cresyl Acetate, Para Tolyl Aldehyde, Phenyl Acetaldehyde, Phenyl Ethyl Acetate, Phenyl Ethyl Butyrate, Phenyl Ethyl Formate, Phenyl Ethyl Iso Butyrate, Phenyl Ethyl Propionate, Phenyl Propyl Acetate, Phenyl Propyl Aldehyd, 4-Methyl-2-(2-methyl-1-propenyl)tetrahydropyran, Styralyl Propionate, Terpineol, Terpinolene, Trans-2-Hexenal,

As a further example, a non-limiting list of flavor components is given which are preferably alone or in combination present as matrix volatile substance V₂ in the matrix inclusions I₂ following the preferred selection described above **(*f***_{**L**} lower than 10² Pa ppm):

Hexyl Cinnamic Aldehyde Alpha, Oxacycloheptadec-10-en-2-one, Linalyl Benzoate, Cedrol, Benzyl Cinnamate, Linalyl Cinnamate, Phenyl Ethyl Cinnamate, Para Cresyl Phenyl Acetate, Benzyl Salicylate, Hexyl Salicylate, Phenyl Ethyl Salicylate, Oxacyclohexadecan-2-one.

The core particle inclusions I₁ and the matrix inclusions I₂ comprising core material volatile substances V₁ and matrix volatile substance V₂ which are optionally deposed in or on a water-insoluble core particle carrier C₁ and a matrix carrier C₂, are non-visible by naked eyes. These inclusions are released during the disintegration of the particulate material and adsorb on substrates exposed to said particulate material. Therefore, in the case of washing an improved deposition and sustained release of said volatile substances on said substrates can be achieved.

By carefully selecting the core particle carrier C₁ and the matrix carrier C₂ the long lastingness of the volatile substances like volatile olfactory top notes on fabrics can be improved.

The hydrophobic carrier material C₁ and/or matrix carrier material C₂ can be selected from hydrophobic liquid diluents, waxes, resins, and polymers belonging to one or more of the following categories:
(1) silicones and modified silicones like linear, ramified or crosslinked poly(dialkylsiloxane) and modified poly(dialkylsiloxane) containing polyether, alkyl, and glycoside side chains, as well as silicones bearing quaternized ammonium functions,
(2) polymers obtained by polymerization of monomers containing an activated carbon-carbon double bond like vinyl, styrenyl and acryl monomers, and
(3) polyolefines.

In another preferred embodiment the core particle carrier C₁ and the matrix carrier C₂ are independently alkali-soluble polymers.

Preferably the core particle carrier C₁ or the matrix carrier C₂ are solvents or diluents which are water insoluble such as hydrophobic liquids such as isopropyl myristate, miglyol, mineral oil, dibenzyl ether, etc. Further, a wax, a resin or a polymer obtained by polymerization of monomers containing an activated carbon-carbon double bond like vinyl and acrylic monomers can be used to minimize the evaporation tendency of very volatile olfactory compounds of the volatile substance.

The outer part of the particulate material according to the present invention is a protecting layer of the inner part. Preferably the material of the outer part has hydrophobic, water repellent characteristics. It is a fat, a fatty acid, a hydrophobized silicon dioxide, a silicone, a silicone-based wax, or a mixture thereof, a modified cellulose, a poly(vinyl alcohol-co-vinyl acetate) copolymer with a degree of hydrolysis preferably lower than 85 % or higher than 91 %, a partially lactonized poly(vinyl alcohol-co-acrylic acid) copolymer, a modified poly(vinyl alcohol) bearing aceto acetate groups, or a mixture thereof. Because of its hydrophobic nature, the outer part can also comprise a outer part volatile substance V₃. Adding fragrance/flavor components to the outer part will allow the particulate material to deliver a smell under dry storage conditions. Preferred fragrance components for the outer phase have loss factor lower than 10² Pa ppm.

In a preferred embodiment the outer part material is a hydrogenated vegetable fat having a melting point ranging between 30 and 60°C, preferably between 35 and 45°C, cellulose or cellulose derivatives like ethyl cellulose, as well as film forming synthetic polymers such as poly (vinyl alcohol), poly (vinyl acetate) or (vinyl butyrate), or poly (vinyl pyrrolidon), or vinyl alcohol copolymers.

Furthermore, with the present invention it has been surprisingly found that the said granulates not only improve the stability and retention of the volatile substance at high loading level during storage under high humidity conditions, but also release the volatile substance on a controlled manner in aqueous media. In particular, the duration and time dependence of the release can be controlled by controlling the thickness of the coating and /or by careful selection of core particle substance S₁, the emulsifier E₁, the matrix substance S₂, the emulsifier E₂ as well as the material of the outer part. In the particular case of laundry detergents, it has been specially recognized that the outer part is a prerequisite to ensure high protection of the granulate particle against moisture and chemical degradation in aggressive detergent bases. It has now been specifically recognized that selecting carefully the outer part and matrix materials confers a high protection against humidity while keeping the disintegration process within a period of time commensurate with conventional machine wash cycles.

Optionally, the particulate material according to the invention can be colored with a water-soluble dye.

A particulate material according to the present invention is prepared by fluidizing core particles, spraying the matrix onto the core particles and finally covering the inner part by the outer part.

Core particles with a high load of core particle inclusions I₁ can be prepared either by spray drying, extrusion, compaction or directly by emulsion polymerization. Spray drying is a preferred preparation method.

The core particle is prepared by emulsifying the core particle inclusion I₁ with an aqueous phase consisting of water, hydrophilic core particle substance S₁ and core particle emulsifier E₁. The amount of emulsifier is set in such a way that the core particle inclusion substance I₁ is dispersed in the form of small droplets or particles in the aqueous phase and that the interface between said droplets or particles and the aqueous phase is saturated with said emulsifier E₁ in order to get a stable oil-in-water feed emulsion for spray drying. This is achieved for example at a core particle inclusion to effective emulsifier mass ratio of roughly 25:2 for typically available modified starch emulsifiers. This mass ratio of 25:2 has to be selected as a higher limit in order to get a stable dispersion of small droplets or particles, to achieve optimal initial fragrance retention during spray drying of particles comprising more than 25% by weight of a volatile substance and to minimize the formation of so-called surface oil, i.e. the appearance of free fragrance at the surface of the particulate material.

Spray drying of core particle emulsion is achieved by using a rotating disk or nozzle atomizers like air pressure nozzle or two-fluid nozzles, preferably a rotating disk, into a co-current hot air drying chamber. Suitable equipment for the emulsion preparation is a batch, high shear propeller inserted into a stirring vessel of appropriate volume size. Alternatively a highpressure mixer can be used. The mean droplet size of the core particle inclusion I₁ has to be below 5 µm. This is achieved by applying either a combination of starch based material and sugar with a starch-based emulsifier E₁ or high efficiency encapsulation systems using polymers of synthetic origin, e.g. poly (vinyl alcohol-co-acetate) copolymers, or other suitable emulsifiers and carrier materials.

The granulation step is carried out in common fluidized bed dryers equipped with a product container. The process is started with the fluidization of the either odorized or non-odorized core particles. The matrix is emulsified in water in the same way as described above for the core particle materials. The emulsified matrix is sprayed into the fluidized core particles using a pressure, sonic or a pneumatic nozzle, preferably, a two-fluid nozzle, which is inserted either on the top (top spray), lateral (lateral spray), tangential (tangential spray), or at the bottom (bottom spray) to the fluidized bed, whereas the lateral position of the nozzle is preferred in the present invention.

Surprisingly it has been found that increasing the nozzle pressure over a critical value, while keeping other process parameters constant, circumvents the drawbacks of the prior art. In particular, it has been surprisingly found that high atomization air pressures not only decreases the size of the feed droplet but also drastically narrows the ultimate particle size distribution of said particulate material. Furthermore, it has been surprisingly found that with high atomization air pressures, not only the size distribution of the particulate material can be controlled but also, as a synergistic effect, the retention of very volatile components can be generally enhanced, yielding retention values of volatile substances as high as 95 % in weight.

Hence, under fast agglomeration conditions (continuous process), the atomization air pressure has to be from 0.1 to 3.0 bar, most preferably from 0.5 to 2.5 bar, while, if slow agglomeration is required (batch process), the pressure has to be set between 2.0 to 8.0 bar, most preferably from 4.0 to 6.0 bar.

Therefore, it is possible to obtain a narrow particle size distribution with conventional fluidized bed granulators without restriction. In particular, particle size distribution similar to those obtained with tangential rotor granulators as mentioned in WO 9716078 can be obtained without relying to this particular technology only.

Due to the fact that this process allows a preparation of a particulate material comprising 30 %, preferably 40 to 60 % of a volatile substances and the loss of volatile substances during granulation is less than 15 %, preferably less than 10 % said process is very cost effective.

A preferred embodiment of the present invention is the use of the particulate material according to the present invention for the controlled release of volatile substances from laundry detergent powders during the wash cycle in washing machines. The release profile is characterized by:
- a latency regime of duration t₁, where almost no volatile, e.g. less than 10 % substance is released, and
- a burst-like release regime of duration t₂, where 60 to 70 % of the volatile substance is released, and
- a continuous release regime of duration t₃, where the remaining 25 to 35 % of the volatile substance is released at a constant rate.

The particulate material is furthermore completely disintegrated after duration time t₂, whereas complete disintegration means in the present context that visible material residues remain neither in suspension in the aqueous medium, nor on substrates treated with products containing said particulate material. In particular, by properly selecting the core particle substance S₁, the emulsifier E₁, the matrix substance S₂ and the emulsifier E₂, as well as the outer part material, the release properties of the particulate material can be controlled in such a way that 80 to 90 % of the volatile substances are delivered to the wash liquor within 15 to 30 minutes at 40°C, while the disintegration of the granules does not exceed 10 minutes. The amount of a particulate material according to the present invention used in such powder detergent systems are above 0.01 %, generally between 0.05 to 2.5 % by weight of the powder, more typically 0.2 to 1.0 %, depending on the kind of powder detergent system, e.g. either concentrated or standard, and on the nature of the fragrance components.

The particulate material according to the present invention may further preferably be used in consumer products, such as body care products, household product and laundry products.

The controlled release and delivery properties of flavors encapsulated in starch matrices may also advantageously be used in dry soups and sauces, instant products, bakery products (cakes, bread specialties, bread mixes, pancake mixes, cake mixes), savory and meat products, pasta, hot and cold beverages (for instance flavored tea), pharmaceutical applications (powders, capsules and tablets), cereals, chewing gum and confectionery products.

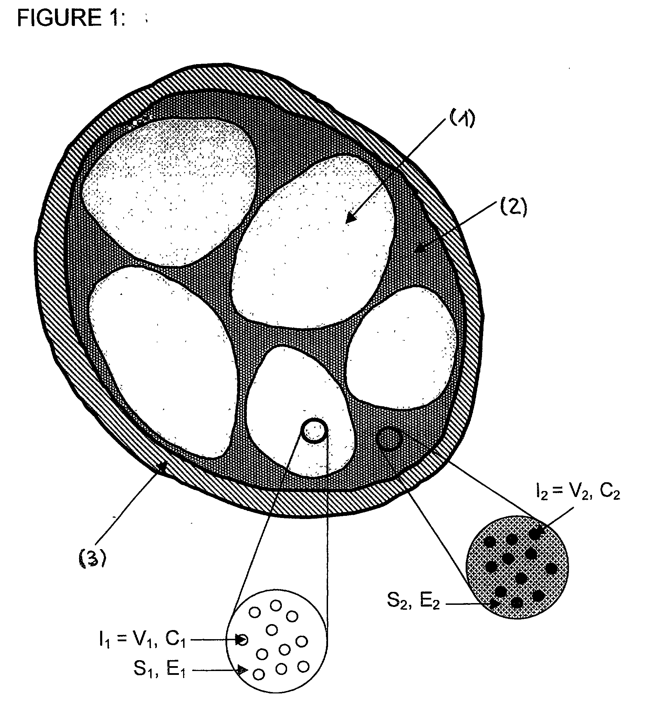

In figure 1 the structure of a preferred embodiment of the particulate material according to the present invention is shown:

The inner part core particles (1) are embedded in a matrix (2). Said inner part is covered by an outer part (3). The core particles (1) comprise a sold, water- or alkali-soluble, or swellable, nonporous core particle substance S1 and an emulsifier E₁. The core particles (1) comprise additionally core particle inclusions I₁. Said core particle inclusions comprise a core particle volatile substance V₁ and core particle carrier C₁. The matrix (2) comprises a matrix substance S₂ and an emulsifier E₂. The matrix comprises additionally matrix inclusions I₂. Said matrix inclusions comprise a matrix volatile substance V₂ and a matrix carrier C₂.

### EXAMPLE 1

The selection of suitable matrix raw materials and composition is achieved by measuring both water content and short-time tack after equilibration of cast films prepared from emulsion of said matrix in controlled atmosphere. This is achieved by (i) applying the emulsified matrix on a glass surface with a calibrated application cylinder in order to get a film thickness of 100 +/- 10 µm (including variance of film thickness due to swelling of the film due to water vapor absorption), (ii) equilibrating the system in climate chambers with known, constant relative humidity for at least 24 hours, (iii) measuring the rolling ball tack, or, alternatively, provided the operator is trained in the art of adhesive formulation, estimating the finger tip grip of the film and (iv) determining the equilibrium water content of the film by any of the conventional water determination techniques like thermogravimetry (TGA), infrared spectroscopy or Karl Fischer titration.

The following table illustrates the selection approach disclosed in the present patent application. The composition of the emulsified matrix, the estimated finger grip tack value (arbitrary units), and the water content at 70% RH and 25°C are listed together with, the particle size distribution parameters (see EXAMPLE 2, below), the bulk density, and the retention of matrix volatile substance V₂ during granulation process (see EXAMPLE 2, below). The results clearly demonstrate the effect of tack and water content on the particle size distribution and initial retention.

| Emulsified matrix composition¹ | Water content at 70% RH and 25°C [wt%]² | Tack³ | Particle | Distribution [µm] | Size | Bulk Density [g/cm³] | Initial Retention HPLC/NMR [%] |
|---|---|---|---|---|---|---|---|
| Example | | | D(v,0.1) | D(v,0.5) | D(v,0.9) | | |
| 2 | 2.2 | 5 | 673 | 1105 | 1722 | 0.70 | 82 |
| 3 | 6.0 | 1 | 711 | 1281 | <3000 | 0.34 | 50⁴ |
| 4 | 3.0 | 5 | 541 | 787 | 1119 | 0.72 | 82 |

| | | | | | | | |
|---|---|---|---|---|---|---|---|
| ¹ Emulsified matrix composition as given in examples. | | | | | | | |
| ² From TGA measurements. | | | | | | | |
| ³ Tack is given in classes: 5 equals to a rolling-ball tack value not exceeding 12 cm, 1 equals to a rolling-ball tack value higher than 30 cm. | | | | | | | |
| ⁴ Normalized to volatile substances V₂ in matrix | | | | | | | |

### EXAMPLE 2

This example describes as a typical recipe example of WO 97/16078 the preparation of a particulate material with a loading of 15% by weight of volatile substance. Unperfumed core particles are used in combination with a medium atomization air pressure prepared by conventional fluidized bed granulation unit.

21.0 kg of coarse sugar were placed as core particles in a conical fluidized bed granulator WS-GT-0.40 (Allgaier Werke GmbH, Germany). The matrix emulsion was prepared by placing 8.63 kg water of 60°C in a separate vessel at room temperature. Thereafter, 5.14 kg of maltodextrin having a dextrose equivalence (DE) of 6 and 7.57 kg of standard coarse sugar, both forming part of the matrix substance S₂, together with 1.55 kg Capsul® (National Starch Ltd., UK) containing matrix emulsifier E₂ and part of the matrix substance S₂ were added and dissolved in said water. 6.72 kg of a standard fragrance oil (Givaudan Vernier SA, Switzerland) forming the matrix volatile substance V₂ are slowly added while stirring vigorously (15500 rpm) with a turbine of a Polyron type (Kinematica AG, Switzerland). The mixture was further homogenized for 10 minutes until a stable emulsion was obtained, e.g. mean droplet size below 1 µm achieved. The emulsified matrix was then sprayed onto the fluidized core particles by applying an atomization air pressure between 2.0 and 3.0 bar with a spraying rate of up to 8.0 kg per hour, whereas the emulsion was atomized by a lateral inserted two-fluid nozzle (Schlick Modell 0/2, Düsen-Schlick GmbH, Germany). The inlet temperature was 65°C and the outlet temperature 53°C. After that, 17.0 kg were removed and the remaining 23.8 kg were coated with 2.5 kg of a vegetable fat having a melting point between 42 to 44°C, which were melted in a water bath of 70 C prior to application. The feed system was pre-heated by means of a manual air heating device. The weight fraction of the vegetable fat related to the theoretical yield of the particulate material was 10.5%. This vegetable fat forms the outer part of said particulate material. The bulk density of the particulate material was 0.70 g/cm³, whereas said material was found dust-free and free-flowing. After a protective screening step (2.0 mm sieve) the particle size was found quite narrow: D(v,0.1) = 673 µm, D(v,0.5) = 1105 µm, D(v,0.9) = 1722 µm, whereas the particle sizes are given as volume average diameter D(v,x) where x denotes the volume fraction of particles having an average diameter below D(v,x). The particulate material disintegrated within 10 min in a stirred standard wash liquor of 40 °C.

The total oil load, measured by standard high pressure liquid chromatography methods (HPLC), was 12 wt% (theoretical perfume load 14.6 wt%) with an initial retention of 82 %.

### EXAMPLE 3

Perfumed core particles were prepared by spray drying on a standard multistage dryer (APV Nordic Anhydro, Denmark) with a water evaporation capacity of 80 kg/h. The core particle emulsion was produced by using 69.3 kg of a maltodextrin having a DE of 6 and forming part of the core particle substance S₁, 18.9 kg of Capsul® (National Starch Ltd., UK), containing core particle emulsifier E₁ and forming part of the core particle substance S₁, 154 kg water, and 37.8 kg of a standard detergent fragrance oil (Givaudan Vernier SA, Switzerland), used in example 2 as matrix volatile substance V₂, constituting the core particle volatile substance V₁. The core particle emulsion was prepared according to standard procedures as described in Example 2. The process parameters where selected such that small agglomeration occurred by choosing an inlet temperature of 190°C, a fluidized bed air inlet temperature of 85°C and a fluidized bed product temperature of 65 °C. The resulting odorized core particles exhibited a total oil load of 30 wt%, as measured by HPLC, resulting in an initial retention of 99%, and a particle size distribution of D(v,0.1)= 81 µm, D(v,0.5)= 154 µm, D(v,0.9)= 258 µm. 6.54 kg of said odorized core particles were placed in a conical fluidized bed granulator WS-GT-0.40 (Allgaier Werke GmbH, Germany). The emulsified matrix was prepared using 2.3 kg of a polyvinyl alcohol, which has a degree of hydrolysation of 88% and a degree of polymerization of 630 and partly forms the matrix emulsifier E₂ as well as the matrix substance S₂, 3.1 kg of standard detergent fragrance oil (Givaudan Vernier SA, Switzerland), used in Example 2 as matrix volatile substance V₂, forming the matrix volatile substance V₂, and 9.3 kg of water, according to procedures described in Example 2. Said emulsified matrix was then sprayed onto the fluidized core particles by applying an atomization air pressure of 5 bar with a spraying rate of up to 11 kg per hour, using the same kind of two-fluid nozzle as used in Example 2. The emulsified matrix was atomized by a lateral inserted two-fluid nozzle. The inlet temperature was 77°C, outlet temperature 55°C. After that, 12.0 kg of the product were covered with 6.7 kg aqueous solution of 20 wt% of the same polyvinyl alcohol as used for the matrix to form the outer part of the particulate material. This yielded in 13.3 kg of particulate material were the dry matter of the outer part related to the theoretical yield of the particulate material was 11 wt%. After screening (2.0 mm sieve), the bulk density was found to be 0.34 g cm⁻³, whereas said particulate material were dust-free and free-flowing. The particle size distribution was quite narrow: D (v,0.1)= 711 µm, D (v,0.5)= 1281 µm, D (v,0.9)= 2019 µm. The particulate material disintegrated within 10 min in a stirred standard wash liquor at 40 °C.

The total oil (measured by HPLC) was 30 wt% (theoretical perfume load 38 wt%) with an initial retention of 79 %.

### EXAMPLE 4

This example describes as a typical recipe example of WO 97/16078 the preparation of a particulate material with a loading of higher than 25% by weight of volatile substance. 14.0 kg of coarse sugar were placed as core material in a conical fluidized bed granulator WS-GT-0.40 (Allgaier Werke GmbH, Germany). The emulsified matrix was prepared according to Example 2 by using 14.3 kg of water, 9.5 kg of Hi-Cap 100® (National Starch Ltd., UK), containing the matrix emulsifier E₂ and part of the matrix substance S₂, 2.4 kg of mannitol and 0.47 kg of triacetin forming part of the matrix substance S₂, and 15.1 kg of a standard detergent fragrance oil (Givaudan Vernier SA, Switzerland), same as used in Example 2, forming the matrix volatile substance V₂. The granulation process was performed according to Example 2 by atomizing the emulsified matrix with a lateral inserted two-fluid nozzle.

| | |
|---|---|
| Air inlet temperature | 60°C |
| Air outlet temperature | 47°C |
| Kind of nozzle | same as in Example 2 |
| Nozzle diameter | 2.0 mm |
| Atomization air pressure | 5.0 bar |
| Feed rate | 1-6 kg/h |

The product was dried for 15 min before cooling down to 30 °C
20.0 kg of the product were put in the product container and coated as in Example 2 with 1.2 kg of a vegetable fat having a melting point between 42 and 44°C by heating said fat to 70°C and spraying said fat onto the product choosing following process parameters:

| | |
|---|---|
| Nozzle configuration | as above |
| Fluidization air | ambient temperature |
| Feed rate | 5 kg/h |

After screening (2.0 mm sieve), a dust-free and free-flowing product was obtained with a bulk density of 0.72 g/cm³. The particle size distribution of said product was quite narrow: D(v,0.1) = 541 µm, D(v,0.5) = 787 µm, D(v,0.9) = 1119 µm.

Additionally, the particulate material had a perfume content of 28 wt% as measured by pulse nuclear magnetic resonance (NMR) relaxation, which measures directly the amount of liquid materials present in the particulate material, with a initial retention of 82 %. The initial perfume profile directly after production (mean initial retention of selected key fragrance components) was measured by GC/MS analysis and found to be also close to 82%. The final coated particulate material disintegrated within 10 min in a stirred standard wash liquor of 40 °C.

### EXAMPLE 5

This example describes a recipe for a typical preparation for the use of large core particles. The spray dried perfumed core particles were produced on a conventional spray dryer Drytec S5; 7" rotary atomizer (Drytec Ltd., UK). Preparation of the feed emulsion was conducted by dissolving 17.0 kg of a polyvinyl alcohol (same as in Example 3), which forms the core particle emulsifier E₁ as well as the core particle substance S₁, in 153.0 kg water at 60°C. After cooling down to ambient temperature, 17.0 kg of a standard detergent fragrance oil (Givaudan Vernier SA, Switzerland), used in Example 2 and constituting the core particle volatile substance V₁, were homogenized as in Example 2. The emulsified matrix was spray dried using the following process parameters:

| | |
|---|---|
| Air inlet temperature | 150 °C |
| Air outlet temperature | 65 °C |
| Feed rate | 50 kg/h |
| Atomizer speed | 18'000 rpm |

The resulting perfumed core particles exhibited a total oil load of 36 %(w/w), as measured by pulsed solid stated NMR, resulting in an initial retention of 72 %. Said capsules had a particle size distribution of D(v,0.1) = 20 µm, D(v,0.5) = 54 µm, D(v,0.9) = 108 µm.

15.0 kg of said perfumed core material were placed in a conical fluidized bed granulator WS-GT-0.40 (Allgaier Werke GmbH, Germany). The emulsified matrix was prepared according to Example 2 by using 12.8 kg of water, 8.5 kg of Hi-Cap 100® (National Starch Ltd., UK) containing the matrix emulsifier E₂ and forming part of matrix substance S₂, 2.1 kg of mannitol and 0.42 kg of triacetin forming part of matrix substance S₂, and 14.6 kg of standard detergent fragrance oil (Givaudan Vernier SA, Switzerland), same as used in Example 2, forming the matrix volatile substance V₂. To enlarge the particle size of the core material, an agglomeration step prior to granulation procedures was performed on said fluidized bed equipment by atomizing 6.5 kg of said emulsified matrix onto said core material using the following process parameters:

| | |
|---|---|
| Air inlet temperature | 80°C |
| Air outlet temperature | 51°C |
| Kind of nozzle | same as in Example 2 |
| Nozzle diameter | 2.0 mm |
| Atomization air pressure | 2.0 bar |
| Feed rate | 1-8 kg/h |

In order to get a narrower particle size distribution, the resulted agglomerated core particles were sieved by means of a 2-deck tumbler screening machine Allgaier TSM 600/2 (Allgaier GmbH, Germany), where the mesh sizes were chosen as 0.8 mm and 0.3 mm. The fraction in-between was used as core particles and the granulation step was performed on said equipment using 8.0 kg of said agglomerated and sieved core material and 31.9 kg of said emulsified matrix by applying following parameters:

| | |
|---|---|
| Air inlet temperature | 65°C |
| Air outlet temperature | 46°C |
| Kind of nozzle | same as in Example 2 |
| Nozzle diameter | 2.0 mm |
| Atomization air pressure | 5.0 bar |
| Feed rate | 1-8 kg/h |

The outer part was applied as in Example 2, whereas 20.0 kg of product were coated with 1.2 kg of a vegetable fat having a melting point between 42 to 44°C. The coating parameters were chosen as follows:

| | |
|---|---|
| Nozzle configuration | same as above |
| Fluidization air | ambient temperature |
| Feed rate | 5 kg/h |

As in previous Examples, the particulate material was sieved by using a mesh size of 2.0 mm, whereas the coated particles had a total oil load of 45 wt% (by NMR) with an initial retention of 91 % related to the granulation and the overall initial retention of 86%. The particle size distribution of said particulate material was found to be as follows: D(v,0.1) = 261 µm, D(v,0.5) = 500 µm, D(v,0.9) = 953 µm, with a bulk density of 0.59 g/cm³, whereas said particulate material disintegrated within 10 min in a stirred standard wash liquor of 40 °C.

### EXAMPLE 6

The following example is considered as a simulation of a continuous process, using the same batch granulator as in Example 2. The first three steps, which include spray drying of core particles, their agglomeration and the sifting step, are normally done simultaneously in one step in equipment designed for continuous agglomeration and granulation. The sieving step simulates the sifting of the product discharge device in the continuous equipment.

Perfumed core particles were produced on a conventional spray dryer Drytec S5; 7" rotary atomizer (Drytec Ltd., UK), similar to previous examples. The core particle emulsion (feed emulsion) was prepared according to homogenization procedures as described in Example 2 using 170 kg of a maltodextrin having a DE of 6 and forming part of the core particle substance S₁, 18.9 kg of Capsul® (National Starch Ltd., UK), containing core particle emulsifier E₁ and forming part of the core particle substance S₁, 230 kg of hot water (70°C), and 81.0 kg of a standard detergent fragrance oil (Givaudan Vernier SA, Switzerland), used in Example 2, constituting the core particle volatile substance V₁. The feed emulsion was spray dried using the following process parameters:

| | |
|---|---|
| Air inlet temperature | 160 °C |
| Air outlet temperature | 90 °C |
| Feed rate | 75 kg/h |
| Atomizer speed | 18'000 rpm |

The resulting perfumed core particles exhibited a total oil content of 25 wt% (initial retention of 83 %) with a particle size distribution of D(v,0.1) = 16 µm, D(v,0.5) = 54 µm, D(v,0.9) = 96 µm.

The emulsified matrix was prepared by procedures described in previous examples using 14.3 kg of water, 9.5 kg of Hi-Cap 100® (National Starch Ltd., UK), which contains the core particle emulsifier E₁ and also forms part of the core particle substance S₁, 2.4 kg of mannitol and 0.2 kg of triacetin forming part of the matrix substance S₂, and 16.3 kg of a standard detergent fragrance oil (Givaudan Vernier SA, Switzerland), same used in Example 2, constituting the matrix volatile substance V₂. 15.0 kg of said odorized core particles were placed in a conical fluidized bed granulator WS-GT-0.40 (Allgaier Werke GmbH, Germany). 21.4 kg of said emulsified matrix was atomized onto said fluidized core particles. The process parameters were chosen as follows:

| | |
|---|---|
| Air inlet temperature | 70°C |
| Air outlet temperature | 45°C |
| Kind of nozzle | same as in Example 2 |
| Nozzle diameter | 1.8 mm |
| Atomization air pressure | 2.0 bar |
| Feed rate | 1-7 kg/h |

In order to simulate the sifting procedure during continuous process, the agglomerated core particles were sieved. After that, the fraction between 0,7 and 1,0 mm was then used as core particles in the following granulation process performed on the same equipment with 9.5 kg of said agglomerated core particles and 21.4 kg of said emulsified matrix. The process parameters were chosen as follows:

| | |
|---|---|
| Air inlet temperature | 65°C |
| Air outlet temperature | 47°C |
| Kind of nozzle | same as in Example 2 |
| Nozzle diameter | 2.2 mm |
| Atomization air pressure | 5.0 bar |
| Feed rate | 1-6.5 kg/h |

The outer part was applied according to previous examples, whereas 15.0 kg of granulated product were coated with 1.5 kg a vegetable fat having a melting point between 42 to 44°C, by applying following process parameters:

| | |
|---|---|
| Nozzle configuration | same as above |
| Fluidization air | ambient temperature |
| Feed rate | 5 kg/h |

The resulting particulate material was sieved using a mesh size of 2.0 mm. The total oil was found to be 40 wt% (by NMR) with an initial retention of 93 %. The particle size distribution was as follows: D(v,0.1) = 231 µm, D(v,0.5) = 460 µm, D(v,0.9) = 979 µm. The bulk density of said particulate material was found to be 0.63 g/cm³, whereas said particulate material disintegrated within 10 min in a stirred standard wash liquor of 40 °C.

### EXAMPLE 7

In this example, it is demonstrated the effectiveness of dividing the perfume into two distinct compositions in order to obtain high perfume load and maximal initial retention. The selection is done such that fragrance components having a loss factor of ***f***_{**L**} > 10² Pa ppm are preferably present in core particle volatile substance V₁. Fragrance components having a loss factor of ***f***_{**L**} < 10² Pa ppm are preferably present in matrix volatile substance V₂. Said particulate material is then compared with a system similar to Examples 2 and 4, where the whole perfume composition is present in the matrix inclusion V₂.

### EXAMPLE 7.1

### Whole perfume in emulsified matrix

14.0 kg of coarse sugar were placed as unperfumed core particle in a conical fluidized bed granulator WS-GT-0.40 (Allgaier Werke GmbH, Germany). The emulsified matrix was prepared according to Example 2 by using 11.3 kg of water, 7.1 kg of Hi-Cap 100® (National Starch Ltd., UK) containing the matrix emulsifier E₂ and forming part of matrix substance S₂, 1.8 kg of mannitol and 0.18 kg triacetin forming part of matrix substance S₂, and 11.3 kg of a standard detergent fragrance oil (Givaudan Vernier SA, Switzerland) forming the matrix volatile substance V₂. The granulation process was performed according to Example 2 with atomizing the emulsified matrix by a lateral inserted two-fluid nozzle.

| | |
|---|---|
| Nozzle diameter | 2.2 mm |
| Kind of nozzle | same as in Example 2 |
| Nozzle air pressure | 5.0 bar |
| Feed rate | 6 - 9.5 kg/h |
| Air inlet temperature | 70 - 78 °C |
| Air outlet temperature | 56 - 65 °C |

The product was dried for 15 min before cooling down to 30 °C.

16.0 kg of the product was put in the product container and coated with 1.6 kg of a vegetable fat having a melting point between 42 and 44°C were heated to 70°C and sprayed onto the product choosing following process parameters:

| | |
|---|---|
| Nozzle configuration | as above |
| Fluidization air | ambient temperature |
| Feed rate | 2.5 - 3 kg/h |

After a protective screening step (2.0 mm sieve) a dust-free and free-flowing product resulted with a bulk density of 0.68 g/cm³. The particle size distribution of said product was quite narrow: D(v,0.1) = 505 µm, D(v,0.5) = 756 µm, D(v,0.9) = 1211 µm.

Additionally, the particulate material had a total oil load of 23 wt% (by NMR) with a initial retention of 79 %. The final particulate material disintegrated within 10 min in a stirred standard wash liquor of 40 °C.

### EXAMPLE 7.2

### Perfume divided into two distinct compositions V₁ and V₂:

The spray dried perfumed core material was produced on a Niro Mobile Minor (Niro A/S, Denmark). Preparation of feed was conducted by dissolving 0.714 kg of maltodextrin MD02 having a DE between 11-14 and forming the core particle substance S₁, and 0.195 kg of Capsul® (National Starch Ltd., UK), which contain the core particle emulsifier E₁ and form part of the core particle substance S₁, in 1.111 kg water at 60°C. After cooling down to ambient temperature 0.390 kg of the core particle volatile substance V₁ of the detergent fragrance oil used in above example were homogenized according to standard procedures similar to Example 2. Eleven of such prepared feed emulsions were spray dried using the following process parameters:

| | |
|---|---|
| Inlet temperature | 180 °C |
| Outlet temperature | 90 °C |
| Feed rate | 4.6 kg/h |
| Atomizer speed | 18'000 rpm |

The eleven spray dried batches were mixed together and the resulting perfumed core particles exhibited a total oil load of 26 % (w/w) (by NMR) resulting in an initial retention of 87 %. Said core particles had a particle size distribution of D(v,0.1) = 11 µm, D(v,0.5) = 29 µm, D(v,0.9) = 77 µm.

8.95 kg of said perfumed core particles were placed in a conical fluidized bed granulator WS-GT-0.40 (Allgaier Werke GmbH). The emulsified matrix was prepared according to Example 2 by using 3.20 kg of water, 1.98 kg of Hi-Cap 100® (National Starch Ltd., UK), which contains the core particle emulsifier E₁ and also forms part of the core particle substance S₁, 0.50 kg mannitol and 0.05 kg triacetin forming part of the matrix substance S₂, and 1.93 kg of the matrix volatile substance V₂. The matrix volatile substance V₂ comprises a selection of fragrance components of the detergent fragance oil (Givaudan Vernier SA, Switzerland) used in Example 7.1 having a loss factor ***f***_{**L**} smaller than 10² Pa ppm, whereas within this Example 7.2 the combination of the two distinct volatile substances for the core particle (V₁) and for the matrix (V₂) form the total detergent fragrance oil used in Example 7.1.

Granulation process was performed on said fluidized bed equipment by atomizing 7.7 kg of said emulsified matrix onto said core particles using the following process parameters:

| | |
|---|---|
| Air inlet temperature | 79 - 85 °C |
| Air outlet temperature | 43 - 53 °C |
| Nozzle diameter (lateral inserted) | 2.2 mm |
| Nozzle pressure | 2.0 bar |
| Feed rate | 2.1- 10.7 kg/h |
| Nozzle type | same as in |
| | Example 2 |

The coating procedure was conducted as in Example 2, whereas 12.0 kg of product was coated with 1.2 kg of a vegetable fat having a melting point between 42 and 44°C pre-heated to 70°C. This time, the process parameters were chosen as follows:

| | |
|---|---|
| Nozzle configuration | same as above |
| Fluidization air | ambient temperature |
| Feed rate | 3.4 kg/h |

As in previous Examples, the particulate material was sieved by using a mesh size of 2.0 mm, whereas the coated particles had a perfume content of 28 wt% (by NMR) with an initial retention of 97 %. The particle size distribution of said product was found to be as follows: D(v,0.1) = 88 µm, D(v,0.5) = 218 µm, D(v,0.9) = 454 µm, with a bulk density of 0.52 g/cm³, whereas said particle disintegrated within 10 min in a stirred standard wash liquor of 40 °C.

The following table demonstrates the effectiveness of dividing the whole perfume into two distinct compositions such as core particle volatile substance V₁ and matrix volatile substance V₂ by following the rules of selecting the fragrance components given in the present invention and in this Example 7. The selection is done such that fragrance components having a loss factor of ***f***_{**L**} > 10² Pa ppm are preferably present in core particle volatile substance V₁. Fragrance components having a loss factor of ***f***_{**L**} < 10² Pa ppm are preferably present in matrix volatile substance V₂. The retention of key fragrances during granulation were measured as in Example 4 by GC/MS analysis.

| Fragrance components | Perfume | | Perfume | Example 7.2 | Example 7.1 | Loss Factor ***f***_{**L**} |
|---|---|---|---|---|---|---|
| | divided into two volatile substance s V₁ (core particle) and V₂ (matrix) | | whole present as matrix volatile substance V₂ | Divided Perfume Initial Retention [%] | Whole Perfume Initial Retention [%] | [Pa ppm] |
| | V₁ | V₂ | V₂ | | | |
| Component A | x | | x | 97 | 48 | 22447 |
| Terpineol | x | | x | 95 | 49 | 15255 |
| Tetrahydro linalool | x | | x | 97 | 47 | 3466 |
| Component B | x | | x | 96 | 49 | 1024 |
| Citronellol | x | | x | 100 | 53 | 761 |
| Aldehyde C10 | x | | x | 93 | 44 | 424 |
| Component C | x | | x | 98 | 7 | 290 |
| Beta ionon | | x | x | 100 | 75 | 90 |
| Dihydro beta ionon | | x | x | 84 | 56 | 84 |
| Component D | | x | x | 84 | 65 | 4.55 |
| Cyclohexyl salicylate | | x | x | 94 | 91 | 1.37 |
| Component E | | x | x | 85 | 75 | 0.55 |
| Benzyl benzoate | | x | x | 88 | 99 | 0.30 |
| Component F | | x | x | 89 | 99 | 0.11 |
| Component H | | x | x | 92 | 100 | 0.09 |

The results demonstrate that the two stage process described in EXAMPLE 7.2, together with the use of the loss factor concept, allows a high recovery of top notes at perfume loads higher than 30%. The composition profile is, therefore, recovered, as is the hedonic attribute of the perfume. This was never achieved in the prior art.

In the following an overview on different product qualities of said particulate materials produced in Example 2 to 7 is given in tabular forms, e.g. particle size, particle size distribution, total oil measured by HPLC or NMR, as well as their initial retention.

| | D(v,0.1) [µm] | D(v,0.5) [µm] | D(v,0.9) [µm] |
|---|---|---|---|
| 2 | 673 | 1105 | 1722 |
| 3 | 711 | 1281 | 2019 |
| 4 | 541 | 787 | 1119 |
| 5 | 261 | 500 | 953 |
| 6 | 231 | 460 | 979 |
| EP 0070719, Example 1^{a} | 254 | 613 | 1093 |
| EP 0070719, Example 2^{a} | 328 | 734 | 1243 |

| | | | |
|---|---|---|---|
| ^{a} calculated using Rosin-Rammler parameters as given in EP 0070719, e.g. Example 1: diameter = 730 µm and n = 2.1, Example 2: diameter = 820 µm and n = 1.9. | | | |

To demonstrated the efficiency of the present invention, a selection of typical particle size distribution from the present invention (Example 4) are given in the figure below. From this figure it can be clearly seen the absence of any severe amounts of dusty materials below D(v) = 100 µm. This is in contrast to the Examples 1 and 2 given in EP 0070719, where the presence of severe amounts of dusty materials can be concluded from the Rosin-Rammler parameters given in said invention. In the figure below, the particle size distributions are plotted versus the normalized volume frequency, defined as the measure (in the case of EP 0070719 calculated) frequency normalized to the half of the frequency of each particle size curve.

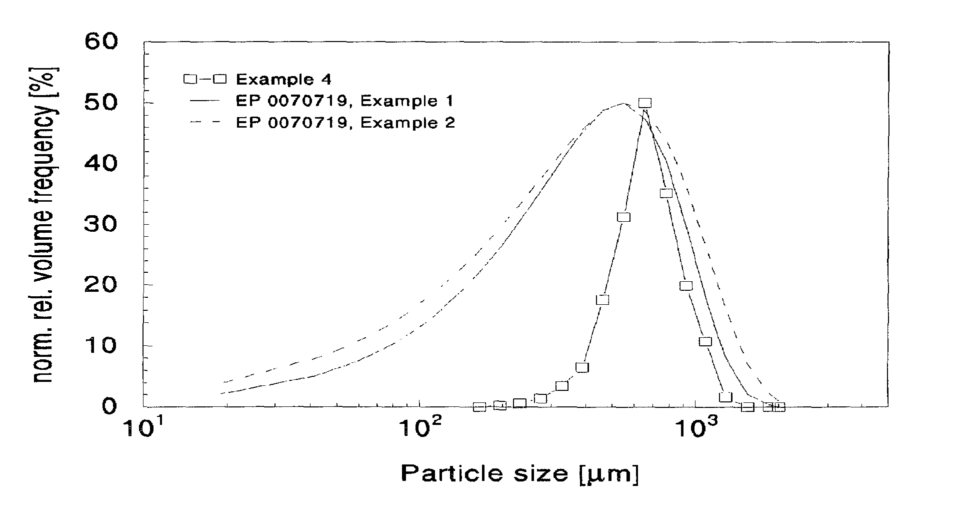

Furthermore, the total oil, initial retention, as well as the initial profile retention, measured by GC/MS methods and given in %, and defined as the mean initial retention of 15-20 selected key fragrance components of the volatile substance (V₁ and/or V₂) used in the present invention are given in the table below.

| Example | Payload theor. [wt%] | Total oil HPLC / NMR [wt%] | Initial Retention [%] | Initial Profile Retention GC/MS [%] |
|---|---|---|---|---|
| 2 | 15 | 12 | 82 | - |
| 3 | 38 | 30 | 79 | - |
| 4 | 34 | 28 | 82 | 82 |
| 5 | 49 | 45 | 91 | 85 |
| 6 | 43 | 40 | 93 | 82 |
| 7.1 | 30 | 23 | 71 | 64 |
| 7.2 | 29 | 28 | 97 | 91 |

### EXAMPLE 8

In this example, a performance of aged particulate material comprising an inner and an outer part compared to particulate material having an inner part of powder detergent is done. The particulate materials were produced as in Example 2, whereas one sample had a outer part applied and the second had no outer part.

Said particulate materials were incorporated into standard, umperfumed, detergent base, resulting in 130.00 g perfumed powder detergent with a perfume level of 0.35 wt% related to the total powder detergent. As a reference, 0.46 g of free perfume were incorporated into 129.54 g of the same standard detergent base. Both samples and reference were filled separately into carton boxes, sealed and stored in a climate chamber at 70 % relative humidity and 37°C for 1 month. All samples were compared in terms of aged perfume profile measured by GC/MS analysis. The results are shown on the Table below

| Fragrance Components | Retention | Retention | Retention |
|---|---|---|---|
| | of aged free perfume [%] | of aged particulate material | of aged particulate material |
| | | with outer part [%] | without outer part [%] |
| Limonene | 0 | 18 | 41 |
| Component A | 32 | 42 | 80 |
| Phenyl ethyl alcohol | 25 | 36 | 72 |
| Tetrahydro linalool | 33 | 50 | 84 |
| Benzyl acetate | 0 | 26 | 50 |
| Citronellol | 26 | 18 | 71 |
| Linalyl acetate | 8 | 39 | 80 |
| Component B | 18 | 41 | 79 |
| Component G | 29 | 53 | 92 |
| Gamma methyl ionone | 49 | 51 | 91 |
| Lilial | 4 | 36 | 65 |
| Hexyl salicylate | 18 | 47 | 94 |
| Component H | 56 | 61 | 104 |
| Component I | 0 | 52 | 94 |
| average | 21 | 41 | 78 |

As it can be seen from the above table, there is a significant improvement of the protection during storage with samples having an outer part.

Standard machine wash tests were performed using following procedures. Terry towels (2.5 kg) were washed in standard wash machines (1 wash cycle, 10 liters water) followed by 2 rinse cycles (27 liters water) at 50°C using 130 g of sample powder described above. Olfactory evaluation was done on wet towels by expert. Results for Example 2 and 3 are given in table below, whereas the scale was as follow: best = 7, worst = 1.

| Samples | Evaluation Example 2 | Evaluation Example 3 |
|---|---|---|
| Aged Reference (0.35% free perfume) | 3.5 | 3.5 |
| Aged Sample (0.35% in particulate material) | 6.0 | 6.5 |
| Fresh Reference (0.35% free perfume) | 7.0 | 7.0 |

The results demonstrate the protective power of the particulate material during storage under high humidity conditions.

### EXAMPLE 9

The deposition of fragrance components on dried towels, washed under the same conditions as in EXAMPLE 8, was measured by thermodesorption followed by a GC/MS analysis. For this example the particulate material of Example 3 was compared to free perfume. Values are given in relative excess deposition percentage, defined as the ratio of the amount of fragrance component deposited from the detergent containing particulate materials by that from the detergent containing the free perfume.

| Key Fragrance Components | relative excess deposition [%] |
|---|---|
| Component B | 366 |
| Lilial | 143 |
| Terpineol | 117 |
| Alpha ionon | 97 |
| Component C | 78 |
| Hexyl salicylate | 75 |
| Benzyl acetate | 60 |
| Component H | 52 |
| Diphenyloxid | 48 |
| Aldehyde C12 MNA | 39 |
| Component J | 15 |
| Eucalyptol | 13 |

These results show that more fragrance components are present on the terry towel after drying, which hints at a much better deposition from the wash liquor. This excess deposition is attributed to the particular fragrance release profile provided by the particulate material during the wash cycle.

### EXAMPLE 10

In this example, the recovery of aged perfume compared to aged particulate material is measured on wet fabrics. Perfumed particulate material from EXAMPLE 2 and aged free perfume were stored and washed as in EXAMPLE 8. Fragrance deposition during the standard machine wash cycle was measured using standard GC/MS SFE analysis techniques. Deposition results are given in wt% of fragrance components deposited onto the fabric related to their initial amount in the wash liquor. The results are given in the table below as an indication of enhanced deposition on wet fabrics.

| Standard detergent fragrance oil | Recovery [wt%] aged perfume | Recovery [wt%] aged particulate material |
|---|---|---|
| Limonene | 0.2 | 0.5 |
| Bornyl acetate | 0.0 | 3.5 |
| Citronellyl acetate | 2.3 | 8.4 |
| Aldehyde C12 MNA | 0.0 | 4.9 |
| Lilial | 0.0 | 5.8 |

Wash methods were similar to Example 8 using terry cotton towels washed with standard procedure at 50°C. In the following a non-limiting selection of applications of said particulate materials as described in the previous examples of the present invention are listed.

### EXAMPLE 11

### Powder Detergent

Large sized particulate material (see Example 2 and 3), optionally colored for visual effects, was mixed with typical powder detergent base at a perfume level of around 0.35 % per weight of the perfumed powder detergent. In the present case, only 30% of the perfume were added as perfumed particulate material, while 70% of the perfume was incorporated as free oil in the unperfumed detergent powder. In parallel, a detergent powder batch was perfumed, according to conventional procedures, with 0.35% perfume as free oil. Both batches were stored at 37 °C and 70% relative humidity for one month.

Substantial benefits of the particulate material compared to conventional systems are a higher storage stability of the perfume leading to smaller losses of perfume, controlled release of the protected fragrance components during the wash cycle and enhanced deposition of these fragrance components on the fabrics.

Additionally, colored particulate material may be used for marketing claims.

In-use tests performed with a panel of consumers under various domestic wash conditions confirm the hedonic benefits of the particulate material according to the present invention. In particular, the fabrics treated with wash powders containing the particulate material according to the invention were judged more fresh and clean that fabrics treated with the conventional powder.

### Detergent Tablets

A defined amount of smaller sized particulate material (see Example 4 and 5) optionally colored for visual effects are mixed with typical detergent tablet base in a perfuming level of around 0.35 % per weight of the perfumed powder detergent.

Substantial benefits of particulate material compared to conventional systems are a higher storage stability of the perfume against bleaching leading to smaller losses of perfume and an enhanced freshness on the washed textile as well as controlled release during wash cycles to circumvent exhausting losses during the whole wash cycle. Additionally, the perfume is protected against migration into the tablet matrix, preventing dissolution problems. Furthermore, colored particulate material may be used for marketing claims.

### Toilet Tablets

Same principles as mentioned for detergent tablets are applicable for the use of toilet tablets.

### Bath Salts

For personal care applications same principles as mentioned for detergent tablets are applicable for the use of toilet tablets.

Further possible applications are given below in a non-limiting manner as follow: house hold products such as toilet cleaners, abrasives; furthermore candles, cosmetic products, etc.

### Deodorants and antiperspirants

Perfumed particulate material according to one of the preceding examples can be added to non-aqueous deodorants and antiperspirant roll-on compositions. Controlled release of the fragrance components is induced by increasing humidity level in the axilliary space.

### Diapers

Perfumed particulate material according to Example 2 or 4 can be mixed with conventional water absorbing material in diapers. The release of the fragrance can be used as a signal of high humidity in the absorbing material, e.g. due to body fluids.

### Food applications

For food application said particulate material can be used to impart a special effect of highly concentrated flavors or the use of more than one flavor within the same particulate material to give a special sensation feeling clearly distinguishable during consuming like a cooling and sparkling effect together with a rounded fruity impression.

## Claims

1. A particulate material comprising at least 25% by weight of a volatile substance, having an inner part covered by an outer part,
the inner part having core particles embedded in a matrix, wherein
the core particles comprise a solid, water- or alkali soluble or swellable, non-porous core particle substance S₁ and core particle inclusions I₁ comprising a core particle volatile substance V₁
and the matrix comprises a hydrophilic matrix substance S₂ and matrix inclusions I₂ comprising a matrix volatile substance V₂.

2. Particulate material according to claim 1 wherein the core particles comprise additionally a core particle emulsifier E₁.

3. Particulate material according to any of the preceding claims wherein the matrix comprise additionally a matrix emulsifier E₂.

4. Particulate material according to any of the preceding claims wherein the core particle inclusions I₁ comprise a core particle carrier C₁ for the core particle volatile substance V₁ and the core particle volatile substance V₁.

5. Particulate material according to any of the preceding claims wherein the matrix inclusions I₂ comprise a matrix carrier C₂ for the matrix volatile substance and the matrix volatile substance V₂.

6. Particulate material according to any of the preceding claims comprising at least 40% by weight of a volatile substance.

7. Particulate material according to any of the preceding claims wherein the core particle volatile substance V₁ comprises at least 70% of fragrance components with a loss factor ***f***_{**L**} higher than 5 Pa ppm.

8. Particulate material according to claim 7, wherein the core particle volatile substance V₁ comprises at least 70% of fragrance components with a loss factor ***f***_{**L**} higher than 100 Pa ppm.

9. Particulate material according to any of the preceding claims wherein the matrix volatile substance V₂ comprises at least 70% of fragrance components with a loss factor ***f***_{**L**} lower than 100 Pa ppm.

10. Particulate material according to any of the preceding claims wherein the particulate material has a particle size of 0.1 to 2 mm.

11. Particulate material according to any of the preceding claims wherein the core particles have a particle size of 0.05 to 1.0 mm.

12. Particulate material according to any of the preceding claims wherein the core particles comprise up to 70% by weight of the core particle inclusions I₁.

13. Particulate material according to any of the preceding claims comprising up to 70% by weight of the matrix inclusions I₂.

14. Particulate material according to any of the preceding claims wherein the core particle inclusion I₁ comprises 50 to 100% by weight of the core particle volatile substance V₁.

15. Particulate material according to any of the preceding claims wherein the matrix inclusion I₂ comprises 50 to 100% by weight of the matrix volatile substance V₂.

16. Particulate material according to any of the preceding claims wherein E₁ is a modified starch material and the ratio between the core particle inclusion I₁ and the core particle emulsifier E₁ is lower than 25:2(w/w).

17. Particulate material according to claim 16 wherein the ratio between the core particle inclusion I₁ and the core particle emulsifier E₁ is 25:2(w/w).

18. Particulate material to any of the preceding claims wherein the core particle volatile substance V₁, the matrix volatile substance V₂ and the outer part volatile substance V₃ are independently an olfactory compound, bio-active materials, bactericides and bacteriostatic agents, cosmetic agents, highly volatile or reactive liquid compounds.

19. Particulate material according to any of the preceding claims wherein core particle substance S₁ and the matrix substance S₂ are independently selected from the group of carbohydrates, natural or synthetic gums, chitin or cationic polysaccharides, cellulose or cellulose derivatives, polypetides, synthetic water soluble or partially water soluble or degradable polymers, polyelectrolytes, biodegradable (bio)polymers, plasticizers or inorganic materials.

20. Particulate material according to any of the preceding claims wherein the core particle carrier C₁ and the matrix carrier C₂ are independently selected from the group of hydrophobic liquid diluents, waxes, resins, silicones and modified silicones, polyolefines or other synthetic polymers.

21. Particulate material according to any of the preceding claims wherein the outer part comprises a fat, a fatty acid, a hydrophobized silicon dioxide, a silicone, a silicone-based wax or a mixture thereof, a modified cellulose, a poly(vinylalcohol-co-vinyl acetate)copolymer, a partially lactonized poly(vinyl alcohol-co-acrylic acid)copolymer, a modified poly(vinyl alcohol) comprising acetate groups, or a mixture thereof.

22. Particulate material according to claim 20 wherein the outer part material has a melting point between 30 and 60°C.

23. Particulate material according to claim 20 wherein the outer part material has a melting point between 35 and 45°C.

24. Laundry products comprising the particulate material according to any of the preceding claims.

25. Body care products comprising the particulate material according to any of the preceding claims.

26. Household products comprising the particulate material according to any of the preceding claims.

27. Food and beverage products comprising the particulate material according to any of the preceding claims.

28. Process for preparing the particulate material according to claims 1 to 23 by fluidizing the core particles, spraying the emulsified matrix onto the core particles, whereby the emulsified matrix is sprayed with a pressure higher than 2 bar, and coating the inner part with the outer part.
